# EUROPEAN PATENT SPECIFICATION

(11) **EP 1 987 810 B1**
(45) Date of publication and mention of the grant of the patent: **07.06.2017**
(21) Application number: 07708402.8
(22) Date of filing: 15.02.2007
(51) Int. Cl.: A61K 8/49, A61Q 19/00, A61Q 19/08, G01N 33/50, A61K 36/38, A61K 31/352

(54) **EXTERNAL PREPARATION FOR SKIN CONTAINING FLAVANONE DERIVATIVE**
EXTERNE ZUBEREITUNG FÜR DIE HAUT MIT FLAVANONDERIVAT
PREPARATION EXTERNE DE LA PEAU CONTENANT UN DERIVE DE FLAVANONE

(30) Priority: 15.02.2006 JP 2006037647; 30.06.2006 JP 2006181185; 30.06.2006 JP 2006181186; 18.07.2006 JP 2006195034
(43) Date of publication of application: 05.11.2008
(73) Proprietor: Pola Chemical Industries Inc., Shizuoka-shi, Shizuoka 422-8009 (JP)
(72) Inventor: KIDA, Naoko, Yokohama-shi, Kanagawa 244-0812 (JP); TADA, Akihiro, Yokohama-shi, Kanagawa 244-0812 (JP); KANAMARU, Akiko, Yokohama-shi, Kanagawa 244-0812 (JP)
(74) Representative: Thurston, Joanna
(86) International application number: PCT/JP2007/052672
(87) International publication number: WO 2007/094384

(56) References cited:
- EP-A1- 1 104 672
- EP-A2- 0 774 249
- JP-A- 11 315 008
- JP-A- H11 315 008
- JP-A- 2002 029 986
- JP-A- 2002 029 986
- JP-A- 2008 115 111
- JP-B1- S4 815 632
- US-A- 5 665 367
- US-A1- 2003 125 264
- O'LEARY R ET AL: "Fucoidan Modulates the Effect of Transforming Growth Factor (TGF)-beta1 on Fibroblast Proliferation and Wound Repopulation in in Vitro Models of Dermal Wound Repair", CHEMICAL AND PHARMACEUTICAL BULLETIN, PHARMACEUTICAL SOCIETY OF JAPAN, JP, vol. 27, no. 2, 1 February 2004 (2004-02-01), pages 266-270, XP003017133, ISSN: 0009-2363
- MATSUOKA E. ET AL.: 'Otogiriso-zoku Shokubutsu no Seibun Kenkyu (1st report), Otogiriso no Kagaku Seibun ni Tsuite' JOURNAL OF TOHOKU PHARMACEUTICAL UNIVERSITY vol. 51, 2004, pages 41 - 48, XP003017132
- O'LEARY R. ET AL.: 'Fucoidan Modulates the Effect of Transforming Growth Factor (TGF)-beta1 on Fibroblast Proliferation and Wound Repopulation in in Vitro Models of Dermal Wound Repair' BIOL. PHARM. BULL. vol. 27, no. 2, 2004, pages 266 - 270, XP003017133

## Description

### Technical Field

The present invention relates to an external preparation for skin, and more specifically, to an external preparation for skin containing a flavanone, namely farrerol.

The present invention also relates to a method of enhancing the collagen production ability of fibroblast.

### Background Art

The skin is formed of the stratum corneum, epidermis, basal stratum, and dermis. It is said that the structure of the dermis has a large effect on the properties of the "skin" which can be commonly recognized. It is also said that one of the important constituents of the dermis is collagen that have a large effect on elastic properties and so on. The collagens of the dermis include collagen I, collagen III, collagen IV, collagen V, collagen VII, and the like and have their own important functions. These collagens are derived from procollagen produced by fibroblasts (see, for example, Non-patent Document 1, Non-patent Document 2, and Non-patent Document 3). A decrease in collagens (including procollagen) production ability of fibroblast is also known to be due to aging and damage caused by UV rays and is responsible for deterioration of skin function. In other words, an important factor to recover the skin function is to restore a collagens production ability of fibroblast from the deterioration.

A person changes in appearance with age and shows aging signs. One of such signs is the formation of wrinkles on the skin, and the major theme of cosmetics is to prevent the formation of such a sign. It has been considered that one of developmental mechanisms of the wrinkle formationmaybe the collapse of the collagen fiber bundle structure of the dermis or the disorder of such a structure. Sometimes, the collapse or disorder of the collagen fiber bundle structure of the dermis leads to a partial lack of the dermis collagen fiber bundle itself. The skin portion in this condition may have a structure similar to that of wounded skin and is popularly termed a "large wrinkle". It has been known that terpene such as monoterpene or triterpene, or a derivative thereof is used for restoring the dermis collagen fiber bundle to improve the collapse or disorder of the collagen fiber bundle structure of the dermis (see, for example, Patent Document 1). In addition, it has been also known that some of plant extracts have the remodeling action on the dermis collagen fiber bundle (see, for example, Patent Document 2 and Patent Document 3). Among the plant extracts, a Hypericum extract has been known to be excellent in remodeling action of the dermis collagen fiber bundle (see, for example, Patent Document 4). These terpenes and extracts have an action to enhance the collagen production ability of fibroblast and, if the collagen fiber bundle is located around, they can build a collagen fiber bundle along such a fiber bundle. However, these substances cannot construct a collagen fiber bundle sufficiently in the case where the dermis collagen fiber bundle itself is lacked and absent around the fibroblasts. Therefore, the above-mentioned terpenes and extracts cannot promote sufficient remodeling of a collagen fiber bundle on a portion lacking collagen fiber bundle, such as a large wrinkle. Therefore, there is a problem in that a sufficient improvement effect on the above-mentioned symptom cannot be exerted.

In addition, the skin is an important tissue for protecting a living body as well as a respiratory organ, so any defect of the skin will threaten both the defense mechanism and the respiratory function of the living body. Thus, there is an established theory that the loss of one-third of the epidermis by suffering burns or the like leads to a danger of life. In this context, the development of a technology for quickly regenerating a defected skin portion has been demanded for many years. Substances with wound-healing action which have been known in the art include, for example, a Curcuma extract and lactoferrin (see, for example, Patent Document 5 and Patent Document 6). However, any of these substances has an insufficient effect of regenerating the dermal tissue because of no remodeling action on the collagen fiber bundle structure of the dermis. In such a circumstance, as substances for wound healing to promote the growth of cells and enhance the collagen productivities thereof, inulins (see, for example, Patent Document 7), collagen hydrolysates (see, for example, Patent Document 8), lactoferrins (see, for example, Patent Document 6), and so on have been developed. The wound-healing action of these substances have been confirmed by means of the regeneration activity of the dermal tissue or the RNA expression of enzymes or the like involved in the proliferative activities of fibroblasts. However, any phenomenon evidently leading to actual skin regeneration by these substances is not observed at all. Further, as for these substances, any influence on the reconstruction of a defective portion of the living body, such a lack of a collagen fiber bundle structure, caused by wound has not been observed at all.

In this way, any substance having an excellent effect in tissue regeneration, wrinkle improvement, wound healing and so on in the living body tissue with a defective portion due to the lack of the collagen fiber bundle structure or the like has not been found at all.

On the other hand, farrerol is a compound having a flavanone structure and known to be included in various plants, and also the synthetic method thereof is already known (see, for example, Patent Document 9, Patent Document 10, and Patent Document 11). However, any technology for incorporating it into an external preparation for skin has not been known at all. Besides, any remodeling action on the dermis collagen fiber bundle and any wound-healing promoting effect have not been known at all.

Further, the co-cultivation study has revealed that the action of keratinocytes may influence an improvement in collagen production ability of fibroblast (see, for example, Non-patent Document 4). However, any substance influencing keratinocytes to enhance the collagen production ability of fibroblast has not been known.

On the other hand, with reference to the collagen production ability of fibroblast, any of the above-mentioned substances for wound healing such as inulins, collagen hydrolysates, and lactoferrins has been screened as one for enhancing the collagen production ability of fibroblast. However, an improvement in collagen production ability of fibroblast is a necessary but not a sufficient condition for promoting wound healing. The present situation is that there is a substance without any effect of promoting wound healing even having an ability of improving collagen productivity.

In addition, a screening method for an effect of promoting wound healing has been also conceived with reference to the migration activity of fibroblasts (see, for example, Non-patent Document 5). In this method, a collagen gel is prepared using fibroblasts and a defective portion is then formed therein, followed by counting the fibroblasts migrating into the defective portion. However, an improvement in migration ability of fibroblasts is a necessary but not a sufficient condition for promoting wound healing. Therefore, there is an ingredient without any effect of promoting wound healing even if it is screened with respect to the migration ability of fibroblasts.

In other words, these screening methods could not screen a substance having both an effect of improving the collagen production ability of fibroblast and an effect of improving the migration ability of fibroblasts, which are required for promoting wound healing. Under such a circumstance, the development of a technology for obtaining a substance having an excellent wound-healing promoting effect has been demanded.

[Patent Document 1] JP 2002-104921 A
[Patent Document 2] JP 2002-29988 A
[Patent Document 3] JP 2002-29987 A
[Patent Document 4] JP 2002-29986 A
[Patent Document 5] JP 11-199461 A
[Patent Document 6] JP 07-196529 A
[Patent Document 7] JP 2004-501176 A
[Patent Document 8] JP 2003-137807 A
[Patent Document 9] WO 02/092110
[Patent Document 10] EP 122053 A
[Patent Document 11] JP 58-21678 A
[Non-patent Document 1] Keene et al., J. Cell Biol., 104, 611-621 (1987)
[Non-patent Document 2] Shimizu et al., Lab. Invest., Jun., 76(6), 753-63 (1997)
[Non-patent Document 3] Vazquez F. et al, Maturitas, 25, 209-15 (1996)
[Non-patent Document 4] Eming S.A. et al., Hum. Gene Ther., 9(4), 529-39 (1998)
[Non-patent Document 5] Roman O'Leary, Mark Rerek, and Edward John Wood, Biol. Pharm. Bull., 27(2), 266-270 (2004)

### Disclosure of the Invention

The present invention intends to provide an external preparation for skin having an excellent remodeling action of disordered collagen fiber bundle structure of the dermis (hereinafter, simply also referred to as a "collagen fiber bundle"). In particular, the present invention intends to provide a technology for promoting wound healing on a skin defect area typified by a large wrinkle or wound by enhancing a tissue regeneration ability of a living body which is typified by a collagen production ability of fibroblast or the like in the dermis.

Further, the present invention intends to provide a technology for regenerating the dermal tissue. Specifically, the present invention intends to provide a technology for enhancing a collagen production ability of fibroblast by acting on keratinocytes.

The present inventors have searched a substance that can be contained in an external preparation for skin, having effects of enhancing a collagen production ability of fibroblast and promoting wound healing, and finally found out that flavanone derivatives such as farrerol can be excellent in such effects. Besides, the present inventors have also found that the flavanone derivatives can effect on keratinocytes to enhance a collagen production ability of fibroblast. Further, the present inventors have completed the present invention by finding out that cosmetics containing flavanone derivatives have excellent effects of improving wrinkles and dermal medicines for external application containing flavanone derivatives have excellent effects of promoting wound healing.

In addition, the present inventors have completed the present invention by finding out that a substance having an effect on promoting wound healing can be efficiently screened by testing the remodeling action of collagen fiber bundle with a method using a skin wound model in the process of searching a substance having a wound-healing promoting activity.

That is, the present invention is as follows.
[1] The invention relates to the use of an external preparation for skin (hereinafter, also referred to as "an external preparation for skin of the present invention") for improving wrinkles , containing a flavanone derivative represented by the following formula (1)

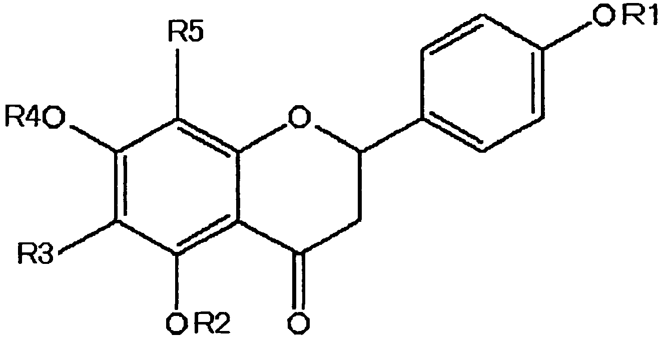

In the formula (1), R1, R2, and R4, each independently represent a hydrogen atom and R3 and R5 each represent an alkyl group with 1 carbon atom.

The flavanone derivative is farrerol.

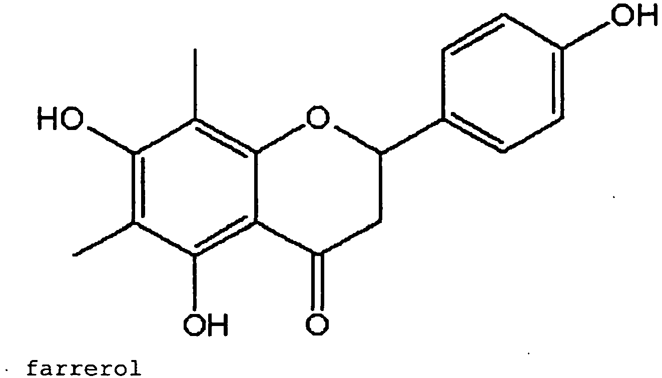

Further, in a preferred embodiment, the external preparation for skin of the present invention contains an extract of *Hypericum erectum, Guttiferae* containing 10⁻⁶ to 0.1% by mass of the flavanone derivative farrerol.

In addition, the external preparation for skin of the present invention can be used for normalizing a skin structure. In particular, it is preferably used for remodeling a disordered dermis collagen fiber bundle. Further, it is preferably used for promoting the regeneration of a skin defect area and the occlusion of the defect area with a regenerated tissue and curing a wound. Besides, it is also preferably used for enhancing the activity of keratinocytes to increase the collagen production ability of fibroblast.

The administration may be transdermal administration. Specifically, the farrerol is preferably contained in an external preparation for skin and then applied to the skin.

### Brief Description of the Drawings

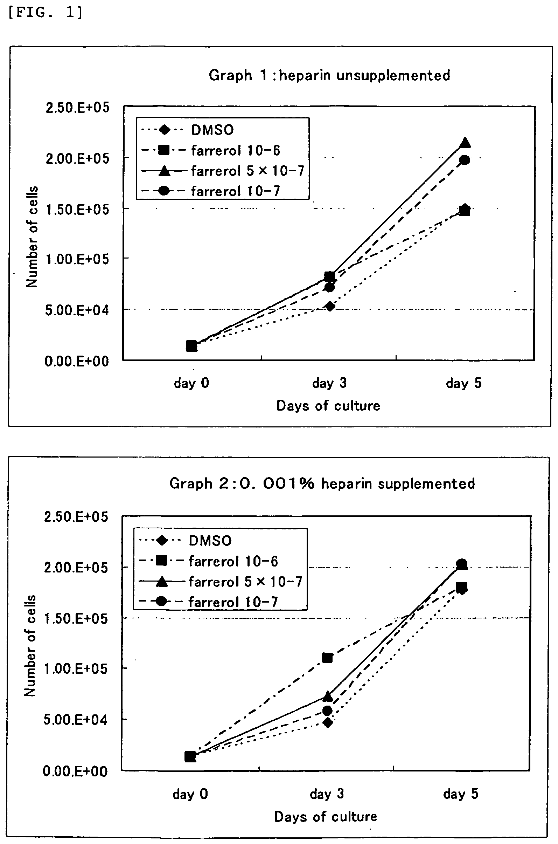

] FIG. 1 is a diagram illustrating the results obtained in Example 1, where graph 1 represents the relation between the number of incubation days and the number of cells in the absence of heparin treatment and graph 2 represents the relation between the number of incubation days and the number of cells in the presence of 0.01%-heparin treatment.
FIG. 2 is a photographic representation of the behavior of fibroblasts observed in a wound-healing model of Example 2.
FIG. 3 is a photographic representation of the behavior of constructing a collagen fiber bundle observed in Example 3.
FIG. 4 is a photographic representation of the state of a collagen-gel-defect area observed in Example 6.
FIG. 5 is a diagram illustrating the results obtained in Experiment 1 of Example 9, where OD values (450 nm) are represented in response to the addition of the respective concentrations of a farrerol solution.
FIG. 6 is a diagram illustrating the results obtained in Experiment 2 of Example 9, where the growth rates of keratinocytes are represented in response to the addition of the respective concentrations of a farrerol solution.
FIG. 7 is a photographic representation of the shape of keratinocytes observed in Experiment 2 of Example 9.
FIG. 8 is a diagram illustrating the results obtained in Experiment 3 of Example 9, where the amount of procollagen produced is represented in response to the addition of the respective concentrations of a farrerol solution.
FIG. 9 is a photographic representation of the state of a collagen-gel-defect area observed in Example 11.

### Best Mode for Carrying out the Invention

The flavanone derivative farrerol can be synthesized from known compounds according to JP 58-21678 A. In addition, it is also present in plants, so it can be also isolated and purified from plant extracts, namely *Hypericum erectum*, Guttiferae. An example of the production process thereof will be described below. Needless to say, when the plant-origin farrerol is contained in an external preparation for skin as described later, an extract containing a given concentration of the farrerol enough to exert a desired effect may be also used.

### <Production Example 1>

10 1 of an aqueous solution of 80% ethanol was added to 1 kg of a dried aerial part of *Hypericum erectum Thunberg* (Guttiferae) and then refluxed for 4 hours while stirring. After cooling, an insoluble matter was removed by filtration and then concentrated under reduced pressure, followed by freeze-drying. Consequently, 51 g of amorphous was obtained. This product was dispersed in water, charged on DIAION HP20, washed with the flows of 5 1 of water and 5 1 of an aqueous solution of 50% ethanol in this order, and eluted with 99% ethanol. The resulting eluate was fractionated. Subsequently, fractions thus obtained are further subjected to thin-layer chromatography and HPLC to checkout the contents thereof to combine the fractions including the same single substance together. On the other hand, the fractions including two or more ingredients are further purified by subjecting them to the chromatography again. The obtained fraction containing the single ingredient is investigated with respect to the action thereof to remodel the collagen fiber bundle. Then, the structure of the single ingredient in the fraction showing such an action strongly was identified using proton NMR, elemental analysis, mass analysis, and ¹³C-NMR. As a result, the ingredient was farrerol with a yield of 3.2 mg. In addition, about 0.006% by mass of the farrerol was included in a solvent-eliminated product which was obtained from the material extracted with an aqueous solution of 80% ethanol.

The above-mentioned flavanone derivative has has an effect of promoting the ability of fibroblasts to remodel the collagen fiber bundle structure of the dermis. Specifically, the flavanone derivative allows fibroblasts to migrate to anywhere even an area where no collagen fiber is present and simultaneously increase the collagen production ability of fibroblast itself, thereby remodeling a collagen fiber bundle on the tissue defect area. Further, wound healing can be promoted by promoting the regeneration of the dermal tissue on the skin defect area and enhancing the ability of the regenerated tissue to occlude the defect area.

### (B) External preparation for skin of the present invention

A characteristic feature of the external preparation for skin of the present invention is to contain the flavanone derivative. For example, it may contain the flavanone derivative as a plant extract. The flavanone derivative is farrerol. Preferably, the external preparation for skin of the present invention may include a *Hypericum erectum* extract containing farrerol.

As described above, the flavanone derivative have an effect of remodeling the collagen fiber bundle structure of the dermis. Thus, the external preparation for skin of the present invention can be used for normalizing the skin structure. Here, the phrase "normalizing the skin structure" means to lower the degree of destructing the structures of the epidermis, the dermis, and the subcutis which constitute the skin. Specifically, for example, it includes the remodeling of a disordered collagen fiber bundle structure of the dermis. Here, the phrase "the remodeling of a disordered collagen fiber bundle structure of the dermis" means to build the collagen fiber bundle structure of the dermis at an area where the dermis collagen fiber bundle structure is disordered. The phrase "the dermis collagen fiber bundle structure is disordered" means that the collagen fiber bundle structure cannot be kept in normal structure. For example, it includes the collagen fiver bundle becomes thin and weak, the amount of the collagen fibers becomes small, the collagen fibers are severed, or the collagen fiber bundle is lacked. Further, the phrase "normalizing the skin structure" also includes the regeneration of skin defect area. It also includes recovering the defect area and promoting the occlusion of the defect area with the regenerated tissue enable to prevent infection from occurring by the defect area and recover the defense mechanism and respiratory function of the skin. Here, the phrase "defect of the skin" means a destroyed state of the skin structure, where, for example, skin roughness, wrinkles, and wound are formed. The external preparation for skin of the present invention is particularly suitable for wound healing. Further, the phrase "normalizing the skin structure" also includes that the activity of keratinocytes to increase the collagen production ability of fibroblast is enhanced.

The external preparation for skin of the present invention can be applied without any specific limitation as long as it is externally applicable to the skin. Examples of the external preparation include cosmetics containing quasi-drugs, dermal medicines for external application, and externally applicable dermatological sundries. Of those, it is particularly preferable to be applied to the cosmetics and the dermal medicines for external application. For example, the cosmetics are preferable for improving wrinkles and mild skin roughness. Specifically, it is preferably used for recovering the dermis collagen fiber bundle from disorder due to photo-aging and for regenerating the collagen fiber bundle structure of the dermis being defected with wound or severe optical damage. In contrast, the dermal medicines for external application are preferable for improving severe skin roughness and for wound healing.

] The dosage form of the external preparation for skin of the present invention is not limited in particular and may be any of dosage forms that the conventional external preparations for skin can be taken depending on the intended use. Preferable examples of the dosage form of the cosmetics include the dosage form to be commonly used for basic skin care such as lotion, milky liquid, essence, cream, and pack. In addition, preferable examples of the dosage form of the external medicines for skin include ointment, lotion, cream, milk, and essence.

The content of the flavanone derivative in the external preparation for skin of the present invention, which can be adjusted depending on the application, target, and administration method of the external preparation for skin, is preferably 1 × 10⁻⁶% by mass and more preferably 1 × 10⁻⁴% by mass as a lower limit. The upper limit is preferably 1% by mass and more preferably 0.1% by mass.

Further, particularly in the case of the dermal medicines for external application to be used for wound healing, the content of the flavanone derivative may be preferably 1 × 10⁻⁶ M and more preferably 1 × 10⁻⁴ M as a lower limit and preferably 1 M and more preferably 0.1 M as an upper limit.

Further, when a plant extract containing the flavanone derivative is contained in the external preparation for skin for the present invention, it is preferable to use one with an increased concentration of the flavanone derivative by purifying the flavanone derivative. The external preparation for skin of the present invention does not include the external preparation for skin to be used for remodeling of the dermis collagen fiber bundle containing a crude *Hypericum erectum* extract and containing no additional flavanone derivative and/or a salt thereof other than one originally included in the extract. The plant extract which can be used in the external preparation for skin of the present invention may be preferably an extract with an increased concentration of farrerol obtained by purification of a plant extract of *Hypericum erectum.* The concentration of the flavanone derivative in the plant extract is 10⁻⁶ to 0.1% by mass and more preferably 10⁻⁵ to 0.01% by mass. In the case of using such a plant extract, the content of the plant extract in the external preparation for skin is preferably 0.001 to 10% by mass and more preferably 0.01 to 1% by mass. Here, the content of the plant extract is synonymous with the content thereof in a solvent-removed product.

The external preparation for skin of the present invention can contain optional ingredients used commonly in an external preparation for skin as well as those essential ingredients. Preferred examples of such an optional ingredient include: oils/waxes such as macadamia nut oil, avocado oil, corn oil, olive oil, rapeseed oil, sesame oil, castor oil, safflower oil, cottonseed oil, jojoba oil, coconut oil, palm oil, liquid lanolin, cured coconut oil, cured oil, Japan wax, cured castor oil, beeswax, candelilla wax, carnauba wax, ibota wax, lanolin, reduced lanolin, hard lanolin, and jojoba wax; hydrocarbons such as liquid paraffin, squalane, pristane, ozokerite, paraffin, ceresin, vaseline, and microcrystalline wax; higher fatty acids such as oleic acid, isostearic acid, lauric acid, myristic acid, palmitic acid, stearic acid, behenic acid, and undecylenic acid; higher alcohols such as cetylalcohol, stearylalcohol, isostearyl alcohol, behenyl alcohol, octyldodecanol, myristyl alcohol, and cetostearyl alcohol; synthetic ester oils such as cetyl isooctanoate, isopropyl myristate, hexyldecyl isostearate, diisopropyl adipate, di-2-ethylhexyl sebacate, cetyl lactate, diisostearyl malate, ethylene glycol di-2-ethyl hexanoate, neopentylglycol dicaprate, glyceryl di-2-heptylundecanoate, glyceryl tri-2-ethylhexanoate, trimethylolpropane tri-2-ethylhexanoate, trimethylolpropane triisostearate, and pentaerythritol tetra-2-ethylhexonate; silicone oil, such as chain polysiloxanes such as dimethylpolysiloxane, methylphenylpolysiloxane, and diphenylpolysiloxane; cyclic polysiloxanes such as octamethylcyclotetrasiloxane, decamethylcyclopentasiloxane, and dodecamethylcyclohexanesiloxane; modified polysiloxanes such as amino-modified polysiloxane, polyether-modified polysiloxane, alkyl-modified polysiloxane, and fluorine-modified polysiloxane; anionic surfactants such as fatty acid soaps (such as sodium laurate and sodium palmitate), potassiumlaurylsulfate, and triethanolamine alkylsulfate ether; cationic surfactants such as trimethyl ammonium stearyl chloride, benzalkonium chloride, and laurylamine oxide; amphoteric surfactants such as imidazoline-based amphoteric surfactants (such as a 2-cocoyl-2-imidazolinium hydroxide-1-carboxyethyloxy disodium salt), betaine-based surfactants (such as alkyl betaine, amide betaine, and sulfo betaine), and acylmethyl taurine; nonionic surfactants such as sorbitan fatty acid esters (such as sorbitan monostearate and sorbitan sesquioleate), glycerin fatty acid esters (such as glycerin monostearate), propyleneglycol fatty acid esters (such as propyleneglycol monostearate), cured castor oil derivatives, glycerol alkyl ether, POE sorbitan fatty acid esters (such as POE sorbitan monooleate and polyoxyethylene sorbitan monostearate), POE sorbitol fatty acid esters (such as POE-sorbitol monolaurate), POE glycerol fatty acid esters (such as POE-glyceryl monoisostearate), POE fatty acid esters (such as polyethyleneglycol monooleate and POE distearate), POE alkyl ethers (such as POE2-octyldodecyl ether), POE alkylphenyl ethers (such as POE nonylphenyl ether), pluronic types, POE/POP alkyl ethers (such as POE/POP2-decyltetradecyl ether), tetronic types, POE castor oil/cured castor oil derivatives (such as POE castor oil and POE cured castor oil), sucrose fatty acid ester, and alkyl glycoside; polyvalent alcohols such as polyethylene glycol, glycerin, 1,3-butylene glycol, erythritol, sorbitol, xylitol, maltitol, propylene glycol, dipropylene glycol, diglycerin, isoprene glycol, 1,2-pentanediol, 2,4-hexanediol, 1,2-hexanediol, and 1,2-octanediol; moisture components such as sodium pyrrolidone carboxylate, lactate, and sodium lactate; fine particles such as mica, talc, kaolin, synthetic mica, calcium carbonate, magnesium carbonate, silicic anhydride (silica), aluminum oxide, and barium sulfate, whose surfaces may be treated; inorganic pigments such as red iron oxide, yellow iron oxide, black iron oxide, cobalt oxide, ultramarine blue, iron blue, titanium oxide, and zinc oxide, whose surfaces may be treated; pearl agents such as mica titanium, fish scale foil, and bismuth oxychloride, whose surfaces may be treated; organic dyes such as Red No. 202, Red No. 228, Red No. 226, Yellow No. 4, Blue No. 404, Yellow No. 5, Red No. 505, Red No. 230, Red No. 223, Orange No. 201, Red No. 213, Yellow No. 204, Yellow No. 203, Blue No. 1, Green No. 201, Purple No. 201, and Red No. 204, which may be laked; organic fine particles such as polyethylene powder, polymethyl methacrylate, nylon powder, and organopolysiloxane elastomer; p-aminobenzoate-based ultraviolet absorbent; an anthranilate-based ultraviolet absorbent; a salicylate-based ultraviolet absorbent; a cinnamate-based ultraviolet absorbent; a benzophenone-based ultraviolet absorbent; a sugar-based ultraviolet absorbent; ultraviolet absorbents such as 2-(2'-hydroxy-5'-t-octylphenyl)benzotriazole, and 4-methoxy-4'-t-butyldibenzoylmethane; lower alcohols such as ethanol and isopropanol; vitamins such as vitamin A or derivatives thereof; vitamin B types such as vitamin B₆ hydrochloride, vitamin B₆ tripalmitate, vitamin B₆ dioctanoate, vitamin B₂ or derivatives thereof, vitamin B₁₂, and vitamin B₁₅ or derivatives thereof; vitamin E types such as α-tocopherol, β-tocopherol, γ-tocopherol, and vitamin E acetate, vitamin D types, vitamin H, pantothenic acid, pantethine, and pyrroloquinoline quinone; and antibacterial agents such as phenoxyethanol.

Of those, the optional ingredient to be contained in the dermal medicines for external application for promoting wound healing is particularly preferably any of ingredients effective to the regeneration of the dermal tissue defected with wound. Specifically, optical ingredient may be particularly preferably, an antibiotic substance for preventing microbial infection such as penicillin, fradiomycin, tetracycline, or a salt thereof, a sterilizer such as acrinol or isodine, or the like. Alternatively, a cicatrization depressant such as tranilast is also preferable. The content of any of these ingredients effective to the regeneration of the dermal tissue is preferably 0.1 to 10% by mass.

The external preparation for skin of the present invention can be produced by processing the flavanone derivative and/or the salt thereof and the optical ingredient by a conventional method.

In addition, the usage of the external preparation for skin of the present invention can be modified depending on the use, dosage form, application site, symptom, and so on.

### ] (C) Method of enhancing action of keratinocytes to increase collagen production ability of fibroblast

A characteristic feature of the method of enhancing the action of keratinocytes to increase the collagen production ability of fibroblasts of the present invention (hereinafter, also referred to as the "method of the present invention") is to administer the flavanone derivative represented by the general formula (1) in the presence of both keratinocytes and fibroblasts.

The flavanone derivative used in the method of the present invention follows the above description of (A). The flavanone derivative have an activity to increase a collagen production ability of fibroblast through an effect on keratinocytes.

The meaning of "collages conceptually includes both collagen, and procollagen which is precursor of collagen.

The phrase "in the presence of both keratinocytes and fibroblasts" means a state in which these cells are coexistent with each other in vitro or in vivo. For example, in vitro, such a state can be produced by co-culture of these cells. In contrast, in vivo, such a state can be found in the skin containing the epidermis and the dermis.

The administration of the flavanone derivative can be performed, for example, by allowing the flavanone derivative to be mixed with a culture medium for co-culture of keratinocytes and fibroblasts. In this case, the amount (dosage) of the mixture is preferably 10⁻⁸ M and more preferably 10⁻⁷ M as a lower limit and preferably 10⁻³ M and more preferably 10⁻⁵ M as an upper limit in terms of the concentration thereof in the medium.

Further, the in vivo administration method may be transdermal administration. The method of transdermal administration is not particularly limited to as long as it is generally used, but preferably a method of applying an external preparation for skin containing the flavanone derivative to the skin. The external preparation for skin which can be used in the method of the present invention is the external preparation for skin of the present invention as described above and a preferred aspect thereof is also formulated as described above.

### Example 1

The remodeling action of farrerol on a dermis collagen fiber bundle was confirmed by an in vitro experiment. In other words, utilizing the fact that, when fibroblasts were cultured in a collagen gel in a heparin supplemented medium, collagen fibers produced from cells were damaged and a strong collagen fiber bundle cannot be formed, the degree of a reduction in damage of the collagen fiber was investigated by allowing farrerol to coexist with the heparin supplemented system. The procedures will be described below.

### <Cells and reagents used>

- Normal human fibroblasts (Kurabo)
- DMEM with 10% FBS (GIBCO)
- WST-8/1-Methyl PMS (Cell Counting Kit-8: DOJINDO LABORATORIES)

Farrerol was dissolved in dimethylsulfoxide (DMSO) and used.

] The toxicity of farrerol was examined by the MTT assay and the dose of farrerol added to the heparin supplemented system was then obtained.

### <Protocol>

1. Fibroblasts grown to 80% confluency were dispersed with 0.05% Trypsin/EDTA. The dispersant was seeded on 96 wells/plate at a concentration of 3 × 10³ cells/well.
2. After 24 hours, farrerol with adjusted concentration was added to the respective wells in a volume of 10 ml/well (n = 8).
3. WST-8/1-Methyl PMS was added to the respective wells in a volume of 10 ml/well after 24 hours, 48 hours, and 72 hours from the addition of farrerol and then incubated at 37°C for 4 hours.
4. Absorbance was measured at a measurement wavelength of 450 nm and a reference wavelength of 650 nm.

### <Results>

Significant cytotoxicity could not be confirmed at any of the concentrations being considered. In contrast, it was confirmed that the addition of farrerol could promote the cell proliferation. In particular, the cell proliferation was promoted when farrerol was at concentrations of 10⁻⁶ ml/ml-DMEM, 5 × 10⁻⁷ ml/ml-DMEM, and 10⁻⁷ ml/ml-DMEM, so the following collagen production test was carried out using these three concentrations.

] On the basis of the above-mentioned results, the action of farrerol to promote the production of collagen was examined using a heparin supplemented system.

### <Cells and reagents used>

- Normal human fibroblasts (Kurabo)
- HEPARIN (SIGMA: H-3149)
- DMEM (GIBCO)
- FBS

### <Protocol>

1. Fibroblasts were grown to confluency in a medium with 10% FBS and then dispersed by trypsin treatment, followed by centrifugation.
2. Supernatant was removed and the fibroblasts were then resuspended in DMEM with 10% FBS, followed by seeding the fibroblasts on a 12-well plate (4.5 cm²/well ) at 3 × 10³ cells/cm².
3. After 4 hours from seeding the cells, the supernatant DMEM was removed and replaced with DMEM with 0.4% FBS, followed by 72-hour incubation under the conditions for preventing cell proliferation (37°C, 5% CO₂).
4. DMEM with 0.4% FBS was removed and then replaced with DMEM with 10% FBS to release the prevention of cell proliferation. In this procedure, the culture conditions were 10% FBS-DMEM, 0.1% heparin/10% FBS-DMEM, and 1.0% heparin/10% FBS-DMEM (each n = 3) for investigating the influence of heparin. Further, those added with farrerol at concentrations of 10⁻⁶ml/ml-DMEM, 5 × 10⁻⁷ ml/ml-DMEM, and 10⁻⁷ ml/ml-DMEM were prepared, respectively.

### <Results>

The results were represented in the graphic diagram of FIG. 1. Graph 1 represents a cell proliferation effect when each concentration of the farrerol solution was added to the medium under the heparin nonsupplemented condition and Graph 2 represents one under the heparin supplemented condition. Any of the conditions indicated that the farrerol-added group tended to promote cell proliferation, compared with the control, DMSO-added group. Consequently, it is found that farrerol has an effect to promote the growth of fibroblasts.

### Example 2

The above-mentioned experiment confirmed that farrerol. had an effect to promote cell proliferation. In addition, for confirming the influence of farrerol on the movement ability of fibroblasts, a "wound-healing model" prepared by punching out a three-dimensional collagen gel by a disposable biopsy punch was used in a comparison between the farrerol-added group and the farrerol-free group with respect to the behavior of fibroblasts derived from the wound area.

### <Cells and reagents used>

- Normal human fibroblasts (Kurabo)
- DMEM with 10% FBS (GIBCO)
- Collagen medium solution (0.5% type-I collagen : 5 × DMEM : 200 mM HEPES : 2.2% NaHCO₃ : 0.1 N NaOH : FBS : water were mixed at a ratio of 4 : 4 : 2 : 2 : 1 : 2 : 3)
- Disposable biopsy punch (3.0 mm in tip diameter)

### <Protocol>

1) The culture of fibroblasts obtained by Protocol 4 of Example 1 and the collagen medium solution were mixed together at a ratio of 1 : 9 (volume) and then suspension-cultured in a 10%-FBS medium for 1 week.
2) When the size of a collagen gel was reduced from one at the time of being prepared by the addition of the collagen medium solution to a size of about 1/5 to 1/10, the center portion of the gel was punched out by a disposable biopsy punch with a diameter of 3 mm.
3) A 6-well plate was prepared and 15 µl of the collagen solution was then dropped on the center of the well. Subsequently, the punched three-dimensional collagen gel was mounted thereon and incubated for 10 minutes (37°C, 5% CO₂).
4) When the dropped collagen was solidified and the three-dimensional collagen gel was attached to the bottom of the well, 10% FBS-DMEM, 0.1% heparin/10% FBS-DMEM, and 1.0%heparin/10% FBS-DMEM were added in a volume of 3 ml/well.
5) Further, a group where 10⁻⁶ ml/ml-DMEM of farrerol was added to each culture condition of (4) was prepared.

### <Results>

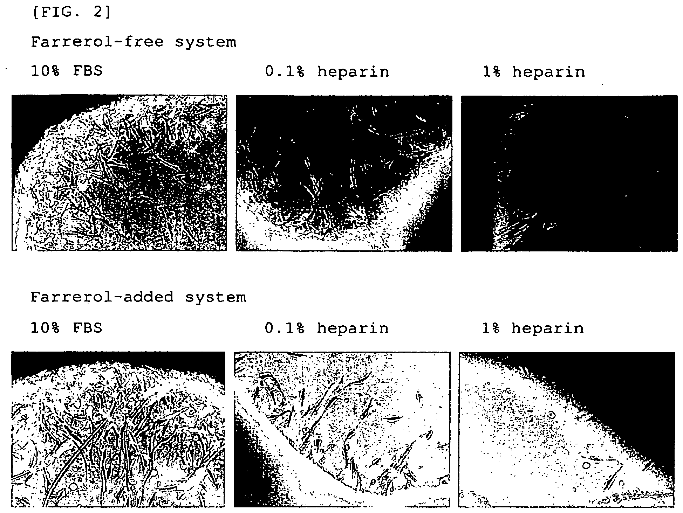

The behavior of fibroblasts after 24 hours from the preparation of the "wound-healing model" was observed. Photographs of fibroblasts derived from the wound area punched out by the disposable biopsy punch were represented in FIG. 2. With respect to the unsupplemented 10% FBS-DMEM, the tendencies of a longer movement distance of fibroblasts and a more active cellular movement were observed in the farrerol-added group, compared with the farrerol-free group. In contrast, with respect to 0.1% heparin/10% FBS-DMEM and 1.0% heparin/10% FBS-DMEM, there was no difference in cell movement between the farrerol-added group and the farrerol-free group and the movement of each of them was prevented by the action of heparin.

Consequently, the results suggest that farrerol does not inhibit the influence of heparin on the movement ability of fibroblasts but directly act on the fibroblasts to enhance the movement ability of the cells.

### Example 3

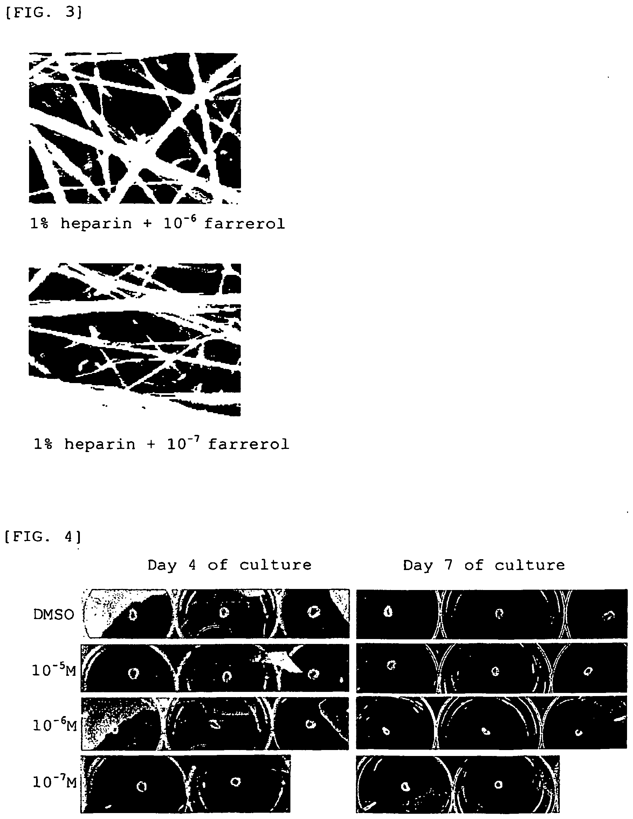

The following procedures were employed to investigate whether farrerol had the action of preventing an inhibitory effect of heparin added on the building of a collagen fiber bundle. The results are represented in FIG. 3. It is found that farrerol prevents an inhibitory effect of heparin on the building of a collagen fiber bundle and exerts a remodeling action of the collagen fiber bundle of the dermis.

### <Protocol>

A collagen solution was prepared by mixture of 0.5% type-I collagen : 5 × DMEM : 200 mM HEPES : 2.2% NaHCO₃ : 0.1 N NaOH : FBS : water at a ratio of 4 : 4 : 2 : 2 : 1 : 2 : 3 (performed while cooling).
↓

A lower-layer collagen solution was prepared by mixing collagen solution with water at a ratio of 9 to 1.
↓

The lower-layer collagen solution was dispensed into the respective wells of a 48-well plate in a volume of 100 µl/well.
↓

Solidification was carried out in a CO₂ incubator (about 15 minutes).
↓

NHDF was recovered according to the routine procedure.
↓

(Cells were separated from one another by a cell strainer).
↓

The number of the cells was counted (Trypan blue) and then adjusted to 1 × 10⁵ cells/ml.
↓

The collagen solution was mixed with the cell suspension at a ratio of 9 to 1 (volume) and then dispensed into the respective wells in a volume of 300 µl/well.

(For preventing the cells fromunevendispersion, the operation proceeded while intermittently stirring (1 × 10⁴ cells/ml at final).
↓

The cells were left standing for 4 hours in the CO₂ incubator.
↓

The gel was separated from the inner wall of the well by an injection needle not to prevent the gel from reduction.
↓

The addition of 1.8% Heparin/10% FBS-DMEM was performed in a volume of 500 µl/well (1% Heparin/well at final).
↓

Farrerol (dissolved in DMSO) was added in a volume of 0.9 µl/well (1,000-fold dilution).
↓

Incubation was carried out in the CO₂ incubator (5 days).
↓

A sample for SEM observation was obtained.

### Example 4

According to the formulation described below, cosmetics (essences) which are the external preparation for skin of the present invention were prepared. A, B, and C were heated to 80°C, B was gradually added to A while stirring to emulsify, and C was then added for neutralization, followed by stirring under cooling. As a result, the essence 1 was obtained. Similar operation was carried out to prepare Comparative Example 1 in which farrerol of essence 1 was substituted with ethanol.

**Table 1**

| Ingredients | % by mass |
|---|---|
| A | |
| Squalane | 5 |
| POE (20) behenyl ether | 1 |
| Behenyl alcohol | 2 |
| Sorbitan sesquistearate | 1.5 |
| Behenic acid | 0.5 |

| B | |
|---|---|
| 1,3-butanediol | 8 |
| 1,2-hexanediol | 4 |
| Phenoxyethanol | 0.5 |
| "Carbopol 1382" | 0.1 |
| (Alkyl-modified carboxyvinyl polymer : manufactured by Goodrich Corporation) | |
| "Pemulen TR-1" | 0.1 |
| (Alkyl-modified carboxyvinyl polymer : manufactured by Goodrich Corporation) | |
| Water | 57.1 |
| 0.1% farrerol solution in ethanol | 0.1 |

| C | |
|---|---|
| Potassium hydroxide | 0.1 |
| Water | 20 |
| Total | 100 |

### <Test Example 1>

Using two groups of 5 individuals, 10 panelists (40 to 50 year old) in total, essence 1 prepared as described above and cosmetics of Comparative Example 1 were subjected to an application test for 8 weeks. These cosmetics were used twice every morning and night on consecutive days and the replicas of the outer corners of the eyes were obtained before and after the application test. Under light irradiation at an oblique angle of 45°, the shadows formed by wrinkles were taken in pictures from a microscope and then binarized to obtain the area ratios of the shadows in the visual field, followed by obtaining the average value thereof in the group. The results are listed in Table 2. Consequently, the cosmetics (essences) which are the external preparation for skin of the present invention are found to have an excellent improvement effect on wrinkles.

| Sample | Average shadowed area ratio (%) | |
|---|---|---|
| | Before test | After test |
| Essence 1 | 6.8±2.3 | 3.3±0.9 |
| Comparative Example 1 | 7.1±2.8 | 5.7±1.6 |

### Example 5

### <Test Example 2>

Essence 2 which is the external preparation for skin of the present invention was prepared according to the following formulation in a manner similar to essence 1. It was evaluated by the method described in Test Example 1. Consequently, an effect was confirmed such that the wrinkle area ratio was changed from 8.2 ± 3.2% before the test to 3.9 ± 1.1% after the test.

**Table 3**

| Ingredients | % by mass |
|---|---|
| A | |
| Squalane | 5 |
| POE (20) behenyl ether | 1 |
| Behenyl alcohol | 2 |
| Sorbitan sesquistearate | 1.5 |
| Behenic acid | 0.5 |

| B | |
|---|---|
| 1,3-butanediol | 8 |
| 1,2-hexanediol | 4 |
| Phenoxyethanol | 0.5 |
| "Carbopol 1382" | 0.1 |
| (Alkyl-modified carboxyvinyl polymer : manufactured by Goodrich Corporation) | |
| "Pemulen TR-1" | 0.1 |
| (Alkyl-modified carboxyvinyl polymer : manufactured by Goodrich Corporation) | |
| Water | 57.1 |
| 0.001% farrerol solution in ethanol | 0.1 |

| C | |
|---|---|
| Potassium hydroxide | 0.1 |
| Water | 20 |
| Total | 100 |

### Example 6

Using a "model of defective dermal tissue with wound" in which a defect area was prepared by punching out a collagen gel, the effect of farrerol to promote the tissue regeneration of a defect area was investigated. The procedures will be described below. The results are represented in FIG. 4. From the figure, it is found that the reduction of a punched hole size is promoted clearly at the final concentration of farrerol of 10⁻⁵ M or 10⁻⁶ M but the promotion of the reduction is unclear at the concentration of 10⁻⁷ M. Consequently, it is found that a lower limit of farrerol is preferably 10⁻⁶ M in the external preparation for skin of the present invention.
1) Normal human fibroblasts grown to 80% confluency were dispersed with 0.05% Trypsin/EDTA. The fibroblasts were seeded at a cell density of 3 × 10⁵ cells/25 cm².
2) After 24 hours, the medium was replaced with one added with farrerol (10⁻⁵ M, 10⁻⁶ M, or 10⁻⁷ M in final concentration) and DMSO, followed by culturing 4 days.
3) A collagen gel was prepared by adding a collagen medium solution to the culture obtained by culturing under each of the conditions in a manner similar to Example 2. The prepared collagen gel was then suspension-cultured for 1 week in the same medium as that of the culture condition of (2). The fibroblasts cultured in the medium with 10⁻⁵ M, 10⁻⁶ M, and 10⁻⁷ M farrerol in (2) were cultured in a medium with 10⁻⁵ M, 10⁻⁶ M, and 10⁻⁷ M farrerol after preparing the collagen gel, respectively.
4) When the size of a collagen gel was reduced from one at the time of being prepared by the addition of the collagen medium solution to a size of about 1/5 to 1/10, the center portion of the gel was punched out in a donut form by a disposable biopsy punch with a diameter of 3 mm. As a result, a "wound model" was prepared.
5) A 6-well plate was prepared and 5 µl of collagen was then dropped on the center portion of the well. Subsequently, the collagen gel of the wound model was mounted thereon and incubated at 37°C for 15 minutes.
6) When the dropped collagen was solidified and the gel was attached to the bottom of the well, 3 ml of the medium with the same condition as that of (3) was added, followed by culturing as a "model of defective dermal tissue with wound". Subsequently, the process of closing up the wound area of the collagen gel was visually observed.

### Example 7

According to the formulation described below, cosmetics (essences) which are the external preparation for skin of the present invention were prepared. A, B, and C were heated to 80°C, B was gradually added to A while stirring to emulsify, and C was then added for neutralization, followed by stirring under cooling. As a result, essence 3 was obtained. Similar operation was carried out to prepare Comparative Example 2 in which farrerol of essence 3 was substituted with ethanol.

**Table 4**

| Ingredients | % by mass |
|---|---|
| A | |
| Squalane | 5 |
| POE (20) behenyl ether | 1 |
| Behenyl alcohol | 2 |
| Sorbitan sesquistearate | 1.5 |
| Behenic acid | 0.5 |

| B | |
|---|---|
| 1,3-butanediol | 8 |
| 1,2-hexanediol | 4 |
| Phenoxyethanol | 0.5 |
| "Carbopol 1382" | 0.1 |
| (Alkyl-modified carboxyvinyl polymer: manufactured by Goodrich Corporation) | |
| "Pemulen TR-1" | 0.1 |
| (Alkyl-modified carboxyvinyl polymer: manufactured by Goodrich Corporation) | |
| Water | 57.1 |
| Material extracted with an aqueous solution of 80% ethanol of Production Example 1 | 0.1 |
| Potassium hydrate | 0.1 |
| Water | 20 |
| Total | 100 |

### <Test Example 3>

Using essence 3 and Comparative Example 2, the effect of promoting the tissue regeneration of a defect area with wound was studied on panelists. A trimming die was put on the medial part of the upper arm of a panelist and the epidermis was stripped off with a scalpel to obtain three parts of 1 mm × 1 mm. The essence 3 was applied to one part once a day for 5 days from the day of removing the epidermis. Comparative Example 2 was applied to another one part once a day for 5 days from the day of removing the epidermis, and the remaining one part was not subjected to any treatment. The observation was performed after 1 week and 2 weeks from the application to determine the degree of tissue regeneration on the defect area. The results are represented in Table 5. Consequently, essence 3, the external preparation for skin of the present invention, is found to be excellent in effect of promoting the tissue regeneration of a defect area. Besides, the formation of scar was not observed at all.

**Table 5**

| Specimen | Observation result after 1 week | Observation result after 2 weeks |
|---|---|---|
| Essence 1 | Most of defect areas are recovered | No difference from normal areas |
| Comparative Example 1 | Scab is present and partial defect remains | Most of defect areas are recovered |
| Untreated | Scab is present and partial defect remains | Most of defect areas are recovered |

### Example 8

### <Test Example 4>

According to the formulation described below, a dermal medicine 1 for external application, the external preparation for skin of the present invention, was prepared in a manner similar to Example 7. The same evaluation as that of Test Example 3 was performed. As a result, the medicine showed an excellent effect of promoting the regeneration of a defect dermal tissue even it coexists with an antibiotic substance.

**Table 6**

| Ingredient | % by mass |
|---|---|
| Squalane | 5 |
| POE (20) behenyl ether | 1 |
| Behenyl alcohol | 2 |
| Sorbitan sesquistearate | 1.5 |
| Behenic acid | 0.5 |
| 1.3-butanediol | 8 |
| 1.2-hexanediol | 4 |
| Phenoxyethanol | 0.5 |
| "Carbopol 1382" | 0.1 |
| (Alkyl-modified carboxyvinyl polymer: manufactured by Goodrich Corporation) | |
| "Pemulen TR-1" | 0.1 |
| (Alkyl-modified carboxyvinyl polymer: manufactured by Goodrich Corporation) | |
| Water | 56.1 |
| Fradiomycin sulfate | 1 |
| Extracted with an aqueous solution of 80% ethanol of Production Example 1 | 0.1 |
| Potassium hydrate | 0.1 |
| Water | 20 |
| Total | 100 |

### Example 9

The effect of farrerol on keratinocytes was studied according the following procedures.

### <Experiment 1: Cytotoxicity evaluation of farrerol on keratinocytes>

### [Cells and reagents used]

- Normal human epidermal keratinocytes (manufactured by Kurabo Co., Ltd.)
- Humedia KG2 (manufactured by GIBCO Co., Ltd.)
- WST-8/1-Methyl PMS (Cell Counting Kit-8: DOJINDO

### LABORATORIES)

### [Sample preparation]

A farrerol sample was prepared by diluting 10⁻² M of farrerol dissolved in DMSO with DMSO to adjust the concentration thereof.

### [Protocol]

1) Normal human epidermal keratinocytes grown to 80% confluency were dispersed with 0.05% Trypsin/EDTA. The dispersant was seeded on 96 wells/plate at a concentration of 3 × 10³ cells/well and 6 × 10³ cells/well.
2) After 24 hours, the farrerol sample with adjusted concentration was added to the respective wells in a volume of 100 µl/well (n = 8).
3) WST-8/1-Methyl PMS was added to the respective wells in a volume of 10 µl /well after 24 hours and 48 hours from the addition of the farrerol sample, and then incubated at 37°C for 4 hours.
4) Absorbance (OD value) was measured at a measurement wavelength of 450 nm and a reference wavelength of 650 nm.

### <Results>

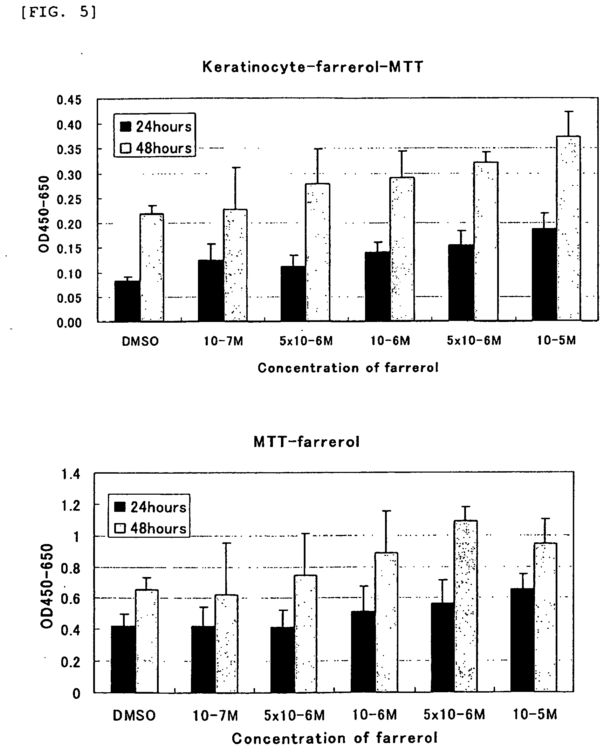

The results of the evaluation with MTT are represented in a graphic diagram of FIG. 5. It was indicated that the OD value increased depending on the concentration of farrerol. For the OD value of 3 × 10³ cells/well at 24 hours, 10⁻⁵M farrerol showed a value two or more times higher than that of the control, DMSO. At 48 hours, it also showed a value 1.7 times higher than that of DMSO. However, the number of the cells was small and the OD value thereof intended to low, so that the reproducibility of the result was confirmed using a doubled number of seeded cells, 6 × 10³ cells/well. As a result, almost the similar result could be obtained.

On the other hand, the visual evaluation did not give the impression that the cell proliferation of the farrerol-added group was promoted compared with that of DMSO.

### ] <Experiment 2: Cell proliferation effect of farrerol on keratinocytes (direct evaluation)>

### [Cells and reagents used]

- Normal human epidermal keratinocytes (manufactured by Kurabo Co., Ltd.)
- Humedia KG2 (manufactured by GIBCO Co., Ltd.)

### [Protocol]

1) Normal human epidermal keratinocytes were prepared at a cell density of 6 × 10³ cells/ml and 1-ml aliquots thereof were then seeded on 96 wells/plate, respectively.
2) After 24 hours, the supernatant was removed from the respective wells and 1 ml of a medium added with a farrerol sample with a concentration adjusted with DMSO was then added to each of the wells.
3) After 24 hours, 48 hours, and 72 hours from the sample addition, the cells were dispersed with 0.05% Trypsin/EDTA and the number thereof was then counted by a blood cell counter. By the way, in Experiment 1, the evaluation for WST-8 was used as an indicator of the number of cells. Since such a method is provided for indirect evaluation of the number of cells by acting on dehydrase in mitochondria, the present study directly count the number of cells.

### [Results]

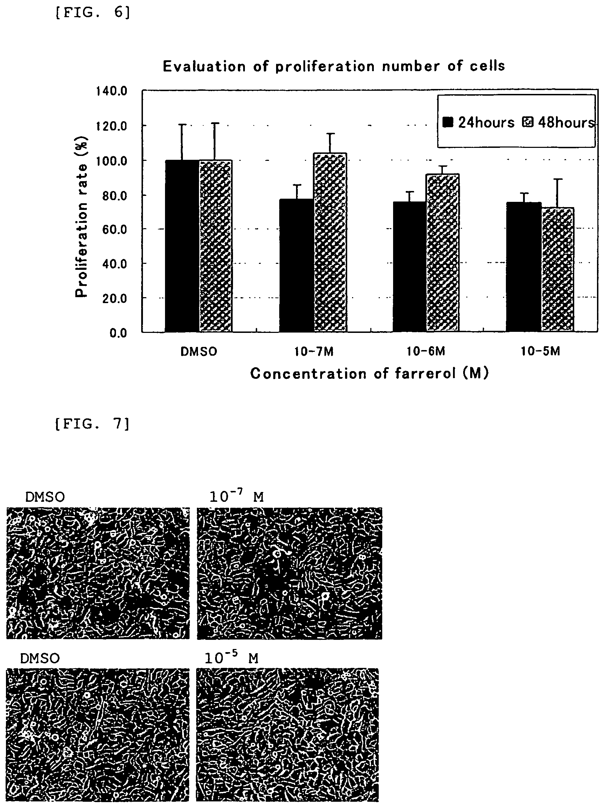

The results of the cell count are represented in FIG. 6. The graph represents the rate of cell proliferation when the number of cells in the control (DMSO-added group) is defined as 100%. Comparing with the control, the cell proliferation rate tended to be inhibited in a concentration-dependent manner. The cell proliferation rate of the 10⁻⁵ M farrerol-added group was inhibited to 75.1 ± 5.2% after 24 hours. In addition, with respect to the other concentrations, the cell proliferation rates were held at 75.5 ± 5.8% with 10⁻⁶ M and 77.1 ± 8.6% with 10⁻⁷ M. After 48 hours, the 10⁻⁵ M farrerol-added group showed a proliferation rate of about 71.9 ± 16.6%. However, at any concentration lower than the above-mentioned concentration showed almost the same proliferation rate as that of the control group. In addition, there was no change in cell shape (FIG. 7). That is, the results of the direct evaluation were not coincident with those of the indirect evaluation on the number of cells with WST-8. As a result, it is implicated that farrerol may promote the reaction of the citric acid cycle in the mitochondria per keratinocyte. Thus, it is expected that the effect of NADH generated during the reaction of the citric acid cycle may also promote a subsequent reaction step, the oxidative phosphorylation reaction. As a result, it is indicated that the generation of ATP provided as an energy source in the cell, or the energy metabolic capacity may be accelerated.

### ] <Experiment 3: Study of influence on fibroblasts through epidermal keratinocytes>

### [Outline]

To find out whether or not any interaction between epidermal keratinocytes and fibroblasts or any effect of farrerol on fibroblasts in coculture could be occurred, an evaluation was performed with reference to the amount of procollagen produced. The method is based on one described in Eming SA et al., Hum. Gene. Ther., 9 (4), 529-39 (1998).

### [Cells and reagents used]

- Normal human epidermal keratinocytes (manufactured by Kurabo Co., Ltd.)
- Humedia KG2 (manufactured by Kurabo Co., Ltd.)
- Normal human fibroblasts (manufactured by Kurabo Co., Ltd.)
- DMEM (manufactured by GIBCO Co., Ltd.)
- PIP EIA Kit (manufactured by TAKARA BIO INC.)

### [Protocol]

### 1) Epidermal keratinocytes

DAY 1: The cells are seeded on a 24-well plate at a cell density of 3 × 10⁴ cells/well.
DAY 6: The plate is washed with PBS and the medium is then replaced with DMEM + 2% FBS, followed by addition of farrerol (10⁻⁶ M, 10⁻⁷ M, 10⁻⁸ M, DMSO).
DAY 7: The supernatant of the culture is collected.

### 2) Fibroblasts

DAY 3: The cells are seeded on a 24-well plate at a cell density of 2.5 × 10⁴ cells/well.
DAY 7: The plate is washed with PBS and the culture supernatant (900 µl) of the epidermal keratinocytes is then added, followed by culturing for 48 hours.
DAY 9: The plate is washed with PBS and serum-free DMEM and the medium is then replaced with 500 µl of serum-free DMEM, followed by culturing for 2 hours. The supernatant of the culture is collected and the amount of procollagen in the centrifuged supernatant (15000 rpm, 5 sec.) was then measured depending on the method described below.

### [Quantitative determination of amount of procollagen produced]

1) After adding 100 µl of a standard antibody solution to each of wells of a plate, a previously-prepared PIP standard solution at each of concentrations (320, 160, 80, 40, 20, and 10 µl/ml; the test substance is diluted with DMEM) and 20 µl of the test sample were added to each well of two rows by means of a micropipette, followed by reacting at 37°C for 3 hours (first reaction).
2) The reaction solution was removed and the wells were then washed with PBS four times. Then, 100 µl of a substrate solution (TMBZ) was added to each well using an eight-tip pipette, followed by reacting at room temperature (20°C to 30°C) for 15 minutes (second reaction).
3) Subsequently, 100 µl of a stop solution was added to each well in the order of the addition of the substrate solution to terminate the reaction and then mixed well.
4) A standard curve was formed by plotting absorbance values at a wavelength of 450 mm while the absorbance of the medium was set to zero as a reference. Then, the PIP concentration was read out from the corresponding absorbance of the test substance.

### [Results]

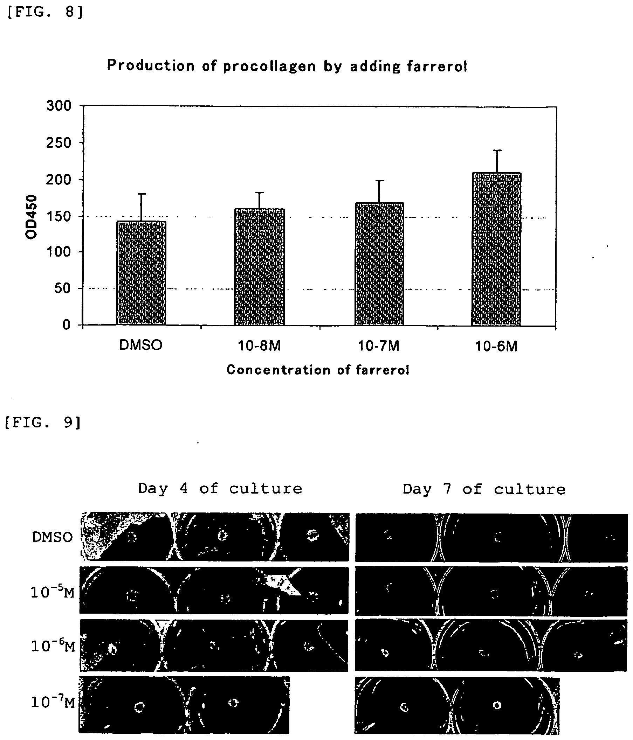

The results are represented in FIG. 8. The amount of procollagen increased significantly depending on the concentration of farrerol. On the other hand, another system in which an evaluation was performed by a method of adding farrerol at the time of culturing fibroblasts and then adding the culture supernatant of epidermal keratinocytes did not show a large change in amount of procollagen produced. From this fact, the effect of farrerol on fibroblasts to directly promote an increase in procollagen production may be small. Consequently, it can be determined that farrerol acts on epidermal keratinocytes and the epidermal keratinocytes promote the effect of fibroblasts to enhance the production of collagens. In this way, the collagen production ability of fibroblast can be changed by using keratinocytes in the coculture system. Thus, for example, with respect to the three-dimensional culture skin or the like, it is applicable to change the properties of three-dimensional skin into desired properties.

### Example 10

A farrerol-containing external preparation for skin (cosmetics: lotion) was prepared according to the formulation described below. The formulated ingredients were mixed and dissolved at room temperature to prepare lotion. The amount of procollagen produced in the dermis of the skin can be increased by transdermally administering the lotion to the skin of a mouse or a human, leading to an increase in amount of collagen in the dermis. This is because keratinocytes are coexistent with fibroblasts in the skin.

**Table 7**

| Ingredients | % by mass |
|---|---|
| 1,3-butanediol | 5 |
| 1,2-pentanediol | 2 |
| 1,2-hexanediol | 3 |
| Glycerine | 5 |
| Phenoxyethanol | 0.5 |
| *Hypericum erectum* extract of Production Example 1 | 0.001 |
| Water | 89.499 |
| Total | 100 |

### Example 11

### <Preparation of test substance>

As a test substance for screening, a *Hypericum erectum* extract was prepared. 10 1 of an aqueous solution of 80% ethanol was added to 1 kg of a dried aerial part of *Hypericum erectum Thunberg* (Guttiferae) and then refluxed for 4 hours while stirring. After cooling, an insoluble matter was removed by filtration and then condensed under reduced pressure, followed by freeze-drying. Consequently, 51 g of amorphous was obtained. This product was dispersed in water, charged on DIAION HP20, washed with the flows of 5 1 of water and 5 1 of an aqueous solution of 50% ethanol in this order, and eluted with 99% ethanol. The resulting eluted fraction was fractionated. Subsequently, fractions thus obtained are further subjected to thin-layer chromatography and HPLC to checkout the contents thereof to combine the fractions including the same single substance together. On the other hand, the fractions including two or more ingredients are further purified by subjecting them to the chromatography again. consequently, seven fractions containing the single ingredient were obtained.

### <Example of screening>

The fractions containing the single ingredients obtained as described above are provided as test substances and the effect of each test substance to construct collagen is then evaluated to screen the substance with a wound-healing effect. The specific procedures will be described later.

Using a "wound model" provided with a defect area formed by punching a collagen gel containing animal fibroblasts, the degree of filling or reduction of the defect area in the presence of a test substance will be observed. The procedures are as follows. The single-ingredient structure of the test substance with the highest degree of filling or reduction of the defect area, compared with the rest of the test substances, was subjected to proton NMR, mass analysis, and ¹³C-NMR and identified as farrerol. By the way, the yield of farrerol from the above extract was 3.2 mg. The content of farrerol in a solvent-removed product of an extract with an aqueous solution of 80% ethanol was about 0.006% by mass.

Further, the final concentration of farrerol was adjusted to each of 10⁻⁵ M, 10⁻⁶ M, and 10⁻⁷ M and the degree of reduction of the defect area at each concentration was then observed. The results are represented in FIG. 9. From the figure, it is found that the reduction of the punch hole (defect area) is distinctly promoted when the final concentration of farrerol is 10⁻⁵M or 10⁻⁶M but the promotion of reduction at 10⁻⁷M is obscure. Consequently, the effect of farrerol to promote the collagen construction can exert a wound-healing promoting effect when at least 10⁻⁶ M of farrerol is used. In this way, according to the screening method of the present invention, the effective concentration of a test substance with a wound-healing promoting effect can be also obtained.

### <Procedures>

1) Normal human fibroblasts grown to 80% confluency in preculture were dispersed with 0. 05% Trypsin/EDTA. The fibroblasts were then seeded on a plate at a cell density of 3 × 10⁵ cells/25 cm² (1 × 10⁴ cells/cm²).
2) After culturing for 24 hours, the medium was replaced with one added with farrerol (10⁻⁵ M, 10⁻⁶ M, or 10⁻⁷ M in final concentration) and DMSO, followed by culturing for 4 days.
3) A collagen gel was prepared by adding a collagen medium solution to the obtained culture in a manner similar to Example 3. The fibroblasts in the collagen gel were cultured using the medium used in (2). The concentration of the test substance in the medium corresponded to the concentration of the medium of (2). That is, the fibroblasts cultured in the medium with farrerol with final concentrations of 10⁻⁵ M, 10⁻⁶ M, and 10⁻⁷ M in (2) were cultured in a medium with farrerol with final concentrations of 10⁻⁵ M, 10⁻⁶ M, and 10⁻⁷ M even after preparing the collagen gel, respectively.
4) After culturing for 1 week, the size of a collagen gel was reduced from one at the time of being prepared by the addition of the collagen medium solution to a size of about 1/5 to 1/10. Subsequently, the center portion of the collagen gel was punched out in a donut form by a disposable biopsy punch with a diameter of 3 mm. As a result, a "wound model" was prepared.
5) A 6-well plate was prepared and 5 µl of the collagen solution was then dropped on the center portion of the well. Subsequently, the collagen gel of the wound model was mounted thereon and incubated at 37°C for 15 minutes, allowing the collagen gel to be attached on the bottom of the well.
6) Subsequently, 3 ml of the same medium as that of (2) was added to each of the wells and the fibroblasts in the above-mentioned collagen gel was then cultured for 7 days. During the culture, the process of closing up the wound area of the collagen gel was visually observed.

### Industrial Applicability

] The external preparation for skin of the present invention is applicable to cosmetics, dermal medicines for external application, and so on. In addition, wound healing can be promoted using the external preparation for skin of the present invention.

Further, with respect to three-dimensional culture skin or the like, the method of enhancing a collagen production ability of fibroblast may be used in the production of artificial skin by changing the properties of three-dimensional skin into desired properties.

A substance with a wound-healing promoting effect suitable for dermal medicines for external application can be screened using the screening method of the present invention.

## Claims

1. Use of an external composition comprising farrerol 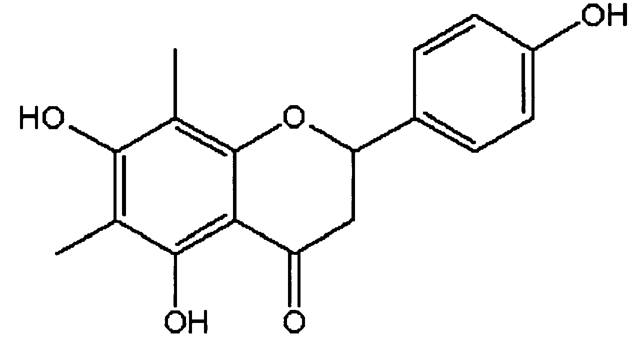 for improving winkles.

2. Use according to claim 1, wherein the composition is an extract of Hypericum erectum, Hypericacae which contains 10⁻⁶ to 0.1% by mass of farrerol.

## Patentansprüche

1. Verwendung einer äußerlich anzuwendenden Zusammensetzung, umfassend Farrerol 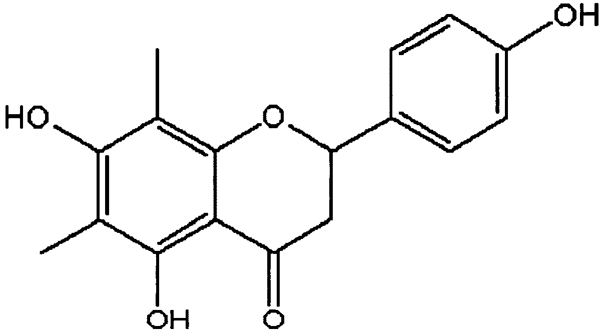 zum Verbessern von Falten.

2. Verwendung nach Anspruch 1, wobei die Zusammensetzung ein Extrakt aus Hypericum erectum, Hypericaceae ist, das 10⁻⁶ bis 0,1 Masseprozent an Farrerol enthält.

## Revendications

1. Utilisation d'une composition à usage externe renfermant du farrerol 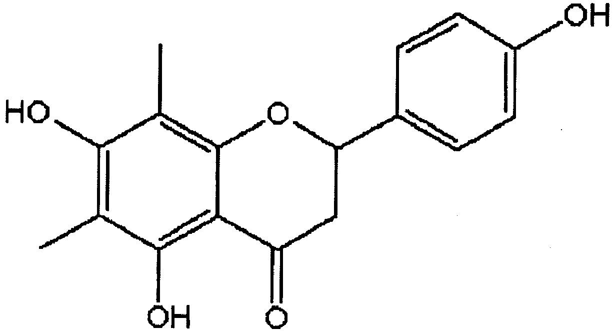 pour diminuer les rides.

2. Utilisation conforme à la revendication 1,
selon laquelle la composition est un extrait d'Hypéricum erectum, Hypericacae qui renferme 10⁻⁶ à 0,1 % en masse de farrerol.
